(19) Europäisches Patentamt / European Patent Office / Office européen des brevets

(11) **EP 4 195 146 B1**

(12) **EUROPEAN PATENT SPECIFICATION**

(45) Date of publication and mention
of the grant of the patent:
**20.11.2024   Bulletin 2024/47**

(21) Application number: **22208695.1**

(22) Date of filing: **22.11.2022**

(51) International Patent Classification (IPC):
*G06T 7/00* (2017.01)          *G06T 7/12* (2017.01)
*G06T 7/149* (2017.01)        *A61B 8/08* (2006.01)
*G16H 50/20* (2018.01)      *G16H 50/30* (2018.01)

(52) Cooperative Patent Classification (CPC):
**A61B 8/085; A61B 8/5223; G06T 7/12;**
**G06T 7/149; G16H 50/20;** G06T 2207/10081;
G06T 2207/10088; G06T 2207/10116;
G06T 2207/10132; G06T 2207/20081;
G06T 2207/30004; G16H 30/40

(54) **ULTRASOUND DIAGNOSTIC APPARATUS AND CONTROL METHOD OF ULTRASOUND DIAGNOSTIC APPARATUS**

ULTRASCHALLDIAGNOSEVORRICHTUNG UND STEUERUNGSVERFAHREN FÜR DIE ULTRASCHALLDIAGNOSEVORRICHTUNG

APPAREIL DE DIAGNOSTIC À ULTRASONS ET PROCÉDÉ DE COMMANDE D'APPAREIL DE DIAGNOSTIC À ULTRASONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **13.12.2021   JP 2021201611**

(43) Date of publication of application:
**14.06.2023   Bulletin 2023/24**

(73) Proprietor: **FUJIFILM Corporation**
**Tokyo 106-8620 (JP)**

(72) Inventor: **IGARASHI, Riki**
**Kanagawa (JP)**

(74) Representative: **Dehns Germany Partnerschaft mbB**
**Theresienstraße 6-8**
**80333 München (DE)**

(56) References cited:
**CN-A- 111 553 919          CN-A- 112 215 842**
**US-A1- 2011 172 533      US-A1- 2012 027 278**
**US-A1- 2017 084 021**

• **GONG L ET AL: "PARAMETRIC SHAPE MODELING USING DEFORMABLE SUPERELLIPSES FOR PROSTATE SEGMENTATION", IEEE TRANSACTIONS ON MEDICAL IMAGING, IEEE, USA, vol. 23, no. 3, 1 March 2004 (2004-03-01), pages 340 - 349, XP001238509, ISSN: 0278-0062, DOI: 10.1109/TMI.2004.824237**

**Description**

**BACKGROUND OF THE INVENTION**

1. Field of the Invention

The present invention relates to a diagnostic apparatus and a control method of the diagnostic apparatus which assist a diagnosis on a subject.

2. Description of the Related Art

In the related art, an image representing a tomogram of an inside of the subject is acquired using a so-called ultrasound diagnostic apparatus or the like, and a user such as a doctor performs an examination for the subject on the basis of the acquired image. In this case, a technique as disclosed in JP2016-202208A has been developed such that the examination for the subject is more accurately performed. JP2016-202208A discloses that an index value relating to visceral fat of a subject is calculated on the basis of a cross-sectional area of the abdomen of the subject and a thickness of subcutaneous fat of the subject measured from an ultrasound image. The cross-sectional area of the abdomen of the subject is approximately measured by approximating the cross section of the abdomen of the subject to an ellipse and calculating the area of the ellipse. US2011172533A1 discloses an apparatus and method which uses an "irregularity degree index" indicating a feature of the boundary line of the liver and outputs the calculated indices in a predetermined form, thereby supporting liver diagnosis.

**SUMMARY OF THE INVENTION**

In order for the user to accurately perform the examination on the subject, as disclosed in JP2016-202208A, not only the area and size of a site of the subject are evaluated, but also the shape of an organ of the subject may be evaluated, such as examination of an organ with an abnormal shape depending on the disease state such as a prostate. In such a case, the user checks an image representing the tomogram of the subject to qualitatively evaluate the shape of the organ in many cases, but in the qualitative evaluation, there are problems that it is difficult to evaluate a change in the shape of the organ in time series, and that accurate evaluation is difficult due to individual differences in the shape of the organ depending on the body type or the like of the subject.

The invention has been made in order to solve such a problem in the related art, and an object of the present invention is to provide a diagnostic apparatus and a control method of the diagnostic apparatus which allow a user to accurately perform an examination on the subject.

According to an aspect of the present invention, there is provided a diagnostic apparatus as claimed in claim 1.

The organ can be a prostate, and the organ evaluation unit can evaluate a disease state of the prostate by evaluating a shape of the prostate on the basis of the shape parameter.

The organ can be a bladder, and the organ evaluation unit can evaluate a disease state of the bladder by evaluating a hollow of the bladder on the basis of the shape parameter.

Further, the image may be an ultrasound image.

In this case, the diagnostic apparatus can further comprise an ultrasound probe; and an image generation unit that generates the ultrasound image by performing transmission and reception of ultrasound beams with respect to the subject using the ultrasound probe.

Further, the image may be any one of an X-ray image, a computed tomography image, or a magnetic resonance image.

According to another aspect, there is provided a control method of a diagnostic apparatus as claimed in claim 7.

**BRIEF DESCRIPTION OF THE DRAWINGS**

Fig. 1 is a block diagram illustrating a configuration of an ultrasound diagnostic apparatus according to an embodiment of the present invention.
Fig. 2 is a block diagram illustrating a configuration of a transmission and reception circuit in the embodiment of the present invention.
Fig. 3 is a block diagram illustrating a configuration of an image generation unit in the embodiment of the present invention.
Fig. 4 is a diagram schematically illustrating an example of an ultrasound image in which a normal prostate is imaged.

Fig. 5 is a diagram schematically illustrating an example of an ultrasound image in which an enlarged prostate is imaged.

Fig. 6 is a diagram illustrating an example in which a shape parameter representing an aspect ratio of a super-ellipse is changed.

Fig. 7 is a diagram illustrating an example in which a shape parameter representing squareness of a super-ellipse is changed.

Fig. 8 is a diagram illustrating an example in which a shape parameter representing deflection of a super-ellipse is changed.

Fig. 9 is a diagram illustrating an example in which a shape parameter representing balance of the shape of a super-ellipse is changed.

Fig. 10 is a diagram illustrating an example of a contour of an organ having a circular shape to be approximated and a curve represented by a basic implicit function used for approximation.

Fig. 11 is a flowchart illustrating an operation of the ultrasound diagnostic apparatus according to the embodiment of the present invention.

Fig. 12 is a diagram illustrating an example of a curve represented by an implicit function.

## DESCRIPTION OF THE PREFERRED EMBODIMENTS

Hereinafter, embodiments of the invention will be described with reference to the accompanying drawings.

The description of configuration requirements described below is given on the basis of the representative embodiment of the present invention, but the present invention is not limited to such an embodiment.

In the present specification, a numerical range represented using "to" means a range including the numerical values before and after "to" as a lower limit value and an upper limit value.

In the present specification, the terms "same" and "identical" include an error range generally allowed in the technical field.

Embodiment

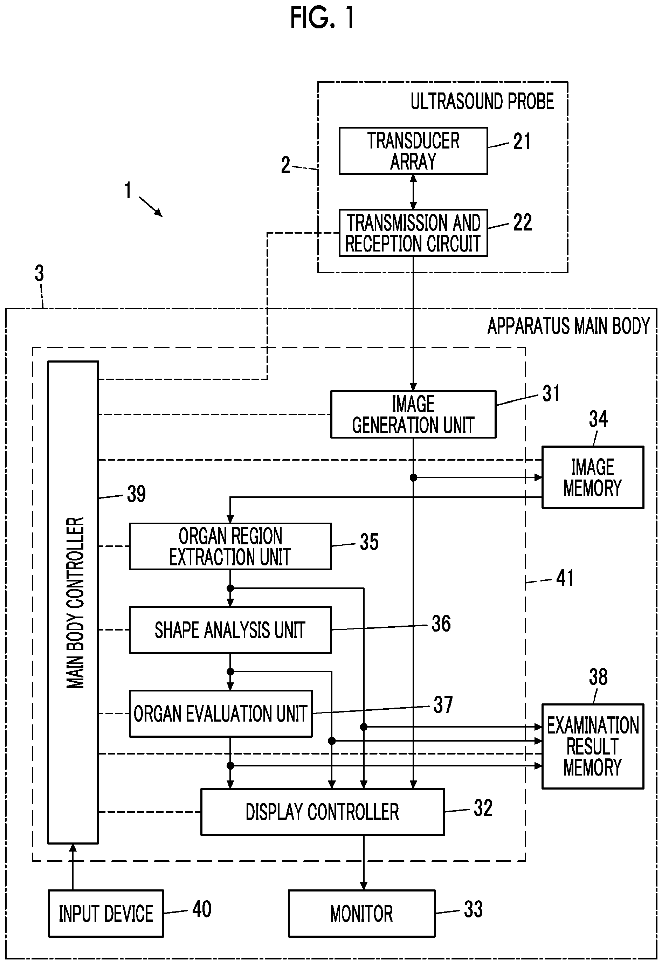

Fig. 1 illustrates a configuration of an ultrasound diagnostic apparatus 1 according to an embodiment of the present invention. The ultrasound diagnostic apparatus 1 comprises an ultrasound probe 2, and an apparatus main body 3 connected to the ultrasound probe 2.

The ultrasound probe 2 comprises a transducer array 21, and a transmission and reception circuit 22 is connected to the transducer array 21.

The apparatus main body 3 comprises an image generation unit 31 connected to the transmission and reception circuit 22 of the ultrasound probe 2. Further, a display controller 32 and a monitor 33 are sequentially connected to the image generation unit 31. An image memory 34 is connected to the image generation unit 31. An organ region extraction unit 35, a shape analysis unit 36, and an organ evaluation unit 37 are sequentially connected to the image memory 34. Further, the organ region extraction unit 35, the shape analysis unit 36, and the organ evaluation unit 37 are connected to the display controller 32. An examination result memory 38 is connected to the image memory 34, the organ region extraction unit 35, the shape analysis unit 36, and the organ evaluation unit 37.

In addition, a main body controller 39 is connected to the transmission and reception circuit 22, the image generation unit 31, the display controller 32, the image memory 34, the organ region extraction unit 35, the shape analysis unit 36, the organ evaluation unit 37, and the examination result memory 38. An input device 40 is connected to the main body controller 39.

Further, the image generation unit 31, the display controller 32, the organ region extraction unit 35, the shape analysis unit 36, the organ evaluation unit 37, and the main body controller 39 constitute a processor 41 for the apparatus main body 3.

The transducer array 21 of the ultrasound probe 2 has a plurality of ultrasonic transducers arranged in a one-dimensional or two-dimensional manner. According to a drive signal supplied from the transmission and reception circuit 22, each of the ultrasonic transducers transmits an ultrasonic wave and receives an ultrasound echo from the subject to output a signal based on the ultrasound echo. For example, each ultrasonic transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

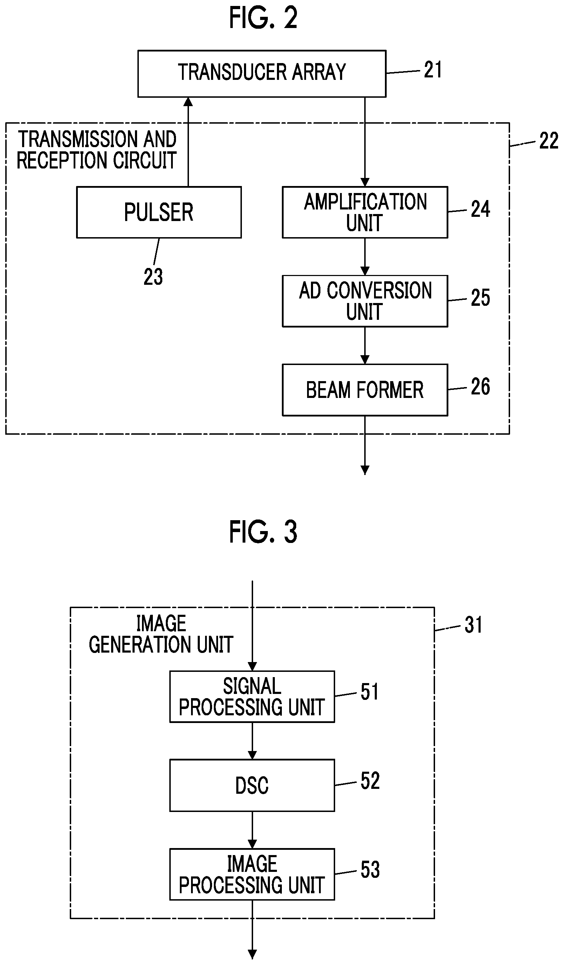

The transmission and reception circuit 22 causes the transducer array 21 to transmit the ultrasonic wave and generates a sound ray signal on the basis of a reception signal acquired by the transducer array 21, under the control of the main body controller 39. As illustrated in Fig. 2, the transmission and reception circuit 22 has a pulser 23 connected to the transducer array 21, and an amplification unit 24, an analog to digital (AD) conversion unit 25, and a beam former 26 that are sequentially connected in series from the transducer array 21.

The pulser 23 includes, for example, a plurality of pulse generators, and the pulser 23 adjusts the amount of delay of each drive signal so that ultrasonic waves transmitted from the plurality of ultrasonic transducers of the transducer array 21 form an ultrasound beam on the basis of a transmission delay pattern selected according to the control signal from the main body controller 39, and supplies the obtained signals to the plurality of ultrasonic transducers. Thus, in a case where a pulsed or continuous-wave voltage is applied to the electrodes of the ultrasonic transducers of the transducer array 21, the piezoelectric body expands and contracts to generate pulsed or continuous-wave ultrasonic waves from each ultrasonic transducer. From the combined wave of these ultrasonic waves, an ultrasound beam is formed.

The transmitted ultrasound beam is reflected by a target, for example, a site of the subject, and propagates toward the transducer array 21 of the ultrasound probe 2. The ultrasound echo propagating toward the transducer array 21 in this manner is received by each ultrasonic transducer constituting the transducer array 21. In this case, each ultrasonic transducer constituting the transducer array 21 expands and contracts by receiving the propagating ultrasound echo to generate a reception signal that is an electric signal, and outputs the reception signal to the amplification unit 24.

The amplification unit 24 amplifies the signals input from each ultrasonic transducer constituting the transducer array 21, and transmits the amplified signals to the AD conversion unit 25. The AD conversion unit 25 converts the signal transmitted from the amplification unit 24 into digital reception data. The beam former 26 performs so-called reception focusing processing in which addition is performed by giving delays to respective pieces of the reception data received from the AD conversion unit 25. Through the reception focusing processing, a sound ray signal in which each piece of the reception data converted by the AD conversion unit 25 is phased and added and the focus of the ultrasound echo is narrowed is acquired.

As illustrated in Fig. 3, the image generation unit 31 has a configuration in which a signal processing unit 51, a digital scan converter (DSC) 52, and an image processing unit 53 are sequentially connected in series.

The signal processing unit 51 generates a B-mode image signal, which is tomographic image information regarding tissues inside the subject, by performing, on the sound ray signal received from the transmission and reception circuit 22, correction of the attenuation due to the distance according to the depth of the reflection position of the ultrasonic wave using a sound speed value set by the main body controller 39 and then performing envelope detection processing.

The DSC 52 converts (raster conversion) the B-mode image signal generated by the signal processing unit 51 into an image signal according to a normal television signal scanning method.

The image processing unit 53 performs various kinds of necessary image processing such as gradation processing on the B-mode image signal input from the DSC 52, and then sends the B-mode image signal to the display controller 32 and the image memory 34. In the following, the B-mode image signal subjected to the image processing by the image processing unit 53 is simply referred to as an ultrasound image.

The main body controller 39 controls the transmission and reception circuit 22 of the ultrasound probe 2 and each unit of the apparatus main body 3 according to a program and the like recorded in advance.

The display controller 32 performs predetermined processing on the ultrasound image or the like generated by the image generation unit 31 and displays the ultrasound image or the like on the monitor 33, under the control of the main body controller 39.

The monitor 33 performs various kinds of display under the control of the display controller 32. The monitor 33 includes a display device such as a liquid crystal display (LCD), or an organic electroluminescence (EL) display.

The input device 40 is for a user to perform an input operation. The input device 40 is configured by, for example, a device for a user to perform an input operation, such as a keyboard, a mouse, a trackball, a touchpad, a touch panel, or the like.

Under the control of the main body controller 39, the image memory 34 stores the ultrasound image generated by the image generation unit 31, and sends the stored ultrasound image to the organ region extraction unit 35. Here, as the image memory 34, for example, recording media such as a flash memory, a hard disk drive (HDD), a solid state drive (SSD), a flexible disk (FD), a magneto-optical disk (MO disk), a magnetic tape (MT), a random access memory (RAM), a compact disc (CD), a digital versatile disc (DVD), a secure digital card (SD card), and a universal serial bus memory (USB memory) can be used.

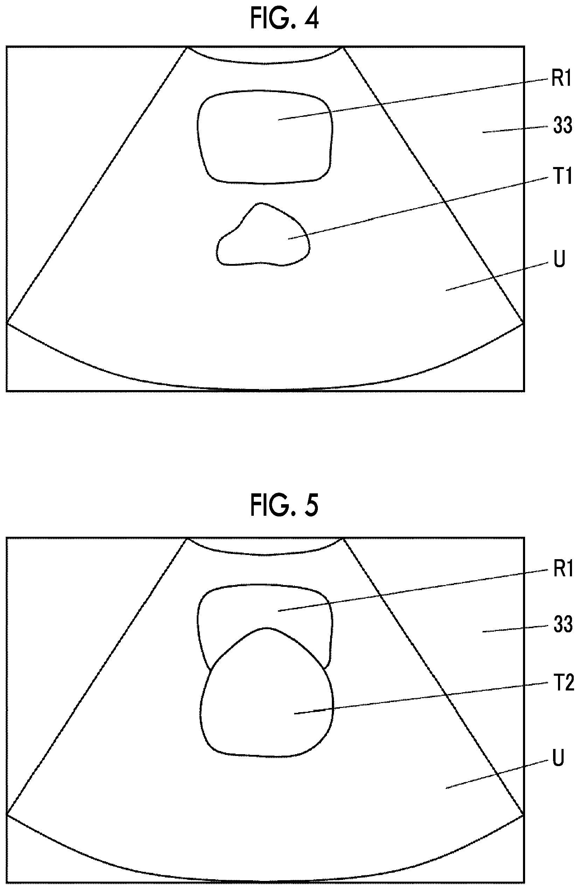

Here, the shape of some organs in the subject, such as the prostate, is changed depending on the disease state. For example, as illustrated in Fig. 4 and Fig. 5, in an ultrasound image U1, an enlarged prostate T2 has a shape relatively close to a circle compared to a normal prostate T1, and is often deformed to protrude toward a bladder R1. In this manner, in a case of examining an organ of which the shape is changed depending on the disease state, usually, the user such as a doctor checks the ultrasound image U to qualitatively evaluate the shape of the organ shown in the ultrasound image U in many cases, but in the qualitative evaluation, there are problems that it is difficult to evaluate a change in the shape of the organ in time series, and that accurate evaluation is difficult due to individual differences in the shape of the organ depending on the body type or the like of the subject.

As described in detail below, in the ultrasound diagnostic apparatus 1 according to the embodiment of the present invention, in order for the user to perform an accurate examination, the ultrasound image U is analyzed to extract an organ included in the ultrasound image U, the extracted organ is approximated by a so-called implicit function, and the disease state of the organ is quantitatively evaluated on the basis of the shape parameter of the approximation curve approximated by the implicit function.

The organ region extraction unit 35 analyzes the ultrasound image acquired from the image memory 34 to extract the organ of the subject. In this case, the organ region extraction unit 35 can extract the organ of the subject included in the ultrasound image by applying a method of using simple pattern matching, a machine learning method described in Csurka et al.: Visual Categorization with Bags of Keypoints, Proc. of ECCV Workshop on Statistical Learning in Computer Vision, pp. 59-74 (2004) or a general image recognition method using deep learning or so-called convolutional neural network (CNN) described in Krizhevsk et al.: ImageNet Classification with Deep Convolutional Neural Networks, Advances in Neural Information Processing Systems 25, pp.1106-1114 (2012).

For example, the organ region extraction unit 35 can display the ultrasound image on the monitor 33 by emphasizing the region of the extracted organ on the ultrasound image. In this case, the organ region extraction unit 35 can display the region of the organ on the ultrasound image in color, display a contour line of the region of the organ on the ultrasound image, display a contour of the region of the organ on the ultrasound image in color, and the like.

The shape analysis unit 36 approximates the shape of the organ extracted by the organ region extraction unit 35 using a so-called implicit function. For example, in a case where an x axis is set along a horizontal direction of the ultrasound image U and a y axis is set along a direction orthogonal to the horizontal direction, the shape analysis unit 36 can use a super-ellipse function indicated by the following Equation (1) as the implicit function.

$$(x/W1)^{\frac{2}{E}} + (y/W2)^{\frac{2}{E}} - 1 = 0 \cdot \cdot \cdot (1)$$

In the super-ellipse function of Equation (1), a position parameter representing an arrangement position or a rotation position of the super-ellipse, a scale parameter representing the size of the super-ellipse, and a shape parameter representing the shape of the super-ellipse can be set. For example, in the function of Equation (1), by replacing the variables x and y with the variables x1 and y1 indicated by Equation (2), the parameters representing the displacement amount of parallel translation of the super-ellipse, that is, the position parameters L1 and L2 representing the arrangement position of the super-ellipse can be set. L1 represents the displacement amount in a direction along the x axis of the super-ellipse, and L2 represents the displacement amount in a direction along the y axis of the super-ellipse.

$$x1 = x - L1, \ y1 = y - L2 \cdot \cdot \cdot (2)$$

Further, by replacing the variables x and y with the variables x2 and y2 indicated by Equation (3), the parameter representing the rotation amount of the super-ellipse, that is, the position parameter A representing the rotation position of the super-ellipse can be set. A satisfies $0 \le A < 2\pi$ ($\pi$ is the circular constant).

$$x2 = x\cos(A) - y\sin(A), \ y2 = x\sin(A) + y\cos(A) \cdot \cdot \cdot (3)$$

In Equation (1), W1 is a scale parameter representing the scale of the super-ellipse in a direction along the x axis, and W2 is a scale parameter representing the scale of the super-ellipse in a direction along the y axis.

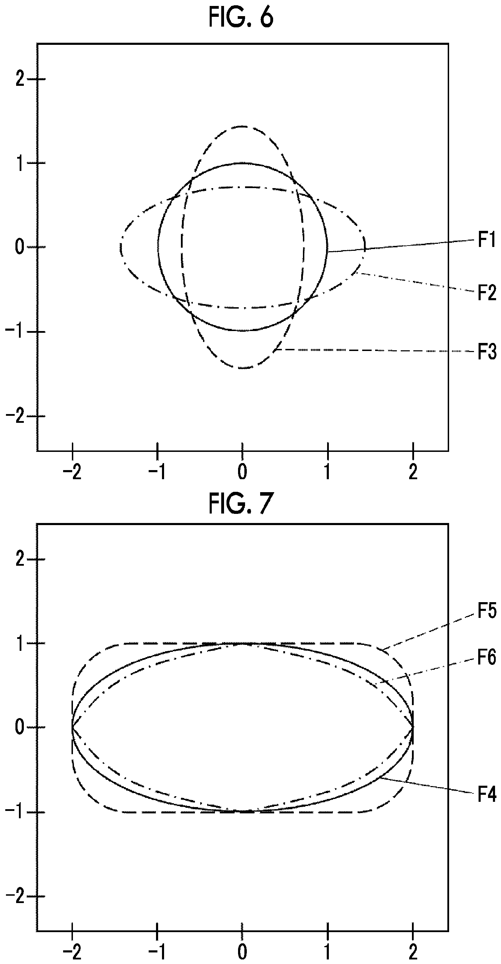

A ratio W1/W2 or W2/W1 between the scale parameters W1 and W2 can be set as the shape parameter representing the aspect ratio of the super-ellipse. For example, Fig. 6 illustrates a curve F1 (circle) in a case where W1:W2 = 1:1, a curve F2 in a case where W1:W2 = 1:2, and a curve F3 in a case where W1:W2 = 2:1, in Equation (1). In this manner, the aspect ratio of the super-ellipse is changed depending on the ratio W1/W2 or W2/W1 between W1 and W2.

Further, E in Equation (1) is a shape parameter representing the squareness of the super-ellipse. Here, the squareness refers to the extent to which the super-ellipse has a shape close to a rectangle, and the super-ellipse is closer to the rectangle as E is larger. Fig. 7 illustrates examples of a curve F4 (ellipse) in a case where E = 1, a curve F5 in a case where E > 1, and a curve F6 in a case where E < 1, in Equation (1). The curve F4 is an ellipse, the curve F5 is a shape closer to a rectangle than an ellipse, and the curve F6 is a shape closer to a rhombus than an ellipse.

Further, by replacing the variables x and y with the variables x3 and y3 indicated by Equation (4), the shape parameter B representing the deflection of the super-ellipse can be set. B satisfies -1 < B < 1 and $B \neq 0$, that is, B satisfies -1 < B < 0 and 0 < B < 1. Further, D in Equation (4) is a variable represented by Equation (5).

$$x3 = (W2/B - y)\sin(D), \ y3 = W2/B - (W2/B - y)\cos(D) \cdot \cdot \cdot (4)$$

$$D = Bx/(W2 - By) \cdot \cdot \cdot (5)$$

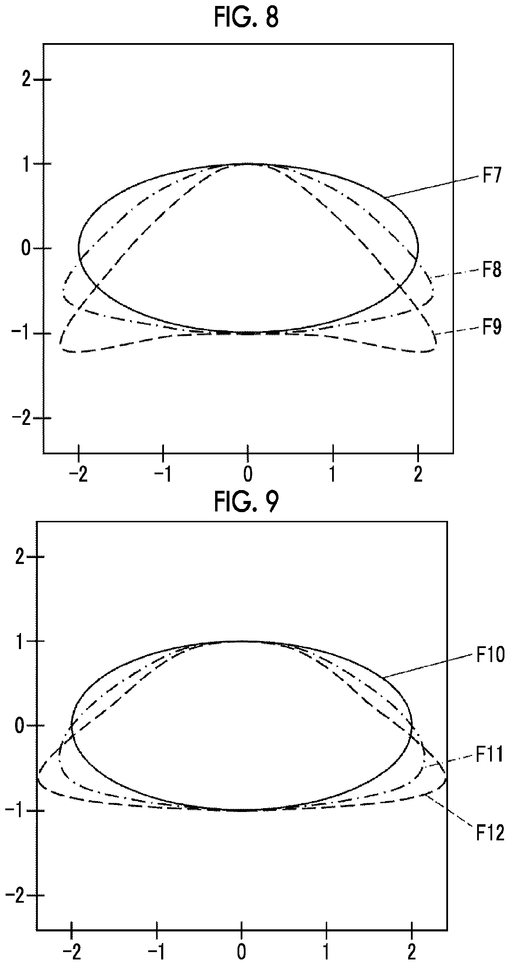

The deflection of the super-ellipse refers to deformation such that both end portions of the super-ellipse in the direction along the x axis are pulled downward, that is, in a negative direction of the y axis. For example, Fig. 8 illustrates a curve F7 (ellipse) without deformation of deflection, a curve F8 in a case where the shape parameter B has a certain value, and a curve F9 in a case where the shape parameter B has a larger value. As described above, the greater the shape parameter B, the greater the deflection of the super-ellipse, and the smaller the shape parameter B, the smaller the deflection of the super-ellipse.

Further, by replacing the variable x with the variable x4 indicated by Equation (6), the shape parameter T representing the balance of the shape of the super-ellipse can be set. T satisfies -1 < T < 1.

$$x4 = (Ty/W2 + 1)x \cdot \cdot \cdot (6)$$

The balance of the shape of the super-ellipse refers to the balance between the width of an upper portion and the width of a lower portion of the super-ellipse, that is, between the width of the positive side portion and the width of the negative side portion of the y axis. For example, Fig. 9 illustrates a curve F10 (ellipse) in a case where the shape parameter T = 0, a curve F11 in a case where the shape parameter T has a certain positive value, and a curve F12 in a case where the shape parameter T has a larger value. As described above, as the shape parameter T is increased in the positive direction, the width of the upper portion of the super-ellipse becomes narrower, and the width of the lower portion of the super-ellipse becomes wider. Although not illustrated, as the shape parameter T is increased in the negative direction, the width of the upper portion of the super-ellipse becomes wider, and the width of the lower portion of the super-ellipse becomes narrower.

In a case where the shape of the organ extracted by the organ region extraction unit 35 is approximated using an implicit function such as a super-ellipse function, the shape analysis unit 36 can perform approximation using an implicit function by applying a so-called steepest descent method, a so-called least squares method, or the like as disclosed in "ZHANG, Xiaoming; ROSIN, Paul L. Superellipse fitting to partial data. Pattern Recognition, 2003, 36.3: 743-752." to the basic implicit function as indicated by Equation (1), for example.

Hereinafter, in order to simply describe the approximation using the implicit function, an example of applying the steepest descent method to the basic implicit function representing the circle indicated by Equation (7) and approximating an organ having a circular shape using the implicit function of Equation (7) will be introduced.

$$(x - a)^2 + (y - b)^2 = r^2 \cdot \cdot \cdot (7)$$

As the approximation procedure, first, an objective function f(a, b, r) indicated by Equation (8) is set using the relationship of Equation (7). Here, $x_i$ and $y_i$ are observation values, that is, coordinates of points set on the contour of the approximated organ. Further, n represents the number of observation values, that is, the number of points set on the contour of the approximated organ. The objective function f(a, b, r) represents the error between n observation values and a, b, and r, and the object of the steepest descent method is to decide the values of a, b, and r so as to minimize the objective function f(a, b, r). By substituting the values of a, b, and r decided in this manner into the implicit function of Equation (7), the implicit function approximating the organ is obtained.

$$f(a, b, r) = \sum_{i=1}^{n} \{(x_i - a)^2 + (y_i - b)^2 - r^2\}^2 \cdot \cdot \cdot (8)$$

Next, using a, b, and r as variables, a partial derivative of the objective function f(a, b, r) with respect to a, a partial derivative of the objective function f(a, b, r) with respect to b, and a partial derivative of the objective function f(a, b, r) with respect to r are calculated, each value is multiplied by a learning rate G, which is a certain value less than 1, and -1, and thereby, change amounts H1, H2, and H3 of a, b, and r for reducing the objective function f(a, b, r) as indicated by Equations (9) to (11) are calculated. Here, initial values are set for each of a, b, and r, and by substituting the initial values into Equations (9) to (11), specific values of the change amounts H1, H2, and H3 are calculated. Further, by adding specific values of H1, H2, and H3 to the initial values of a, b, and r, specific values of a, b, and r that make the objective function f(a, b, r) smaller are newly decided.

$$H1 = -G \ \partial/\partial a \ f(a, b, r) \cdot \cdot \cdot (9)$$

$$H2 = -G \ \partial/\partial b \ f(a, b, r) \cdot \cdot \cdot (1\,0)$$

$$H3 = -G \ \partial/\partial r \ f(a, b, r) \cdot \cdot \cdot (1\,1)$$

By repeating such processing, values of a, b, and r that minimize the objective function f(a, b, r) are decided.

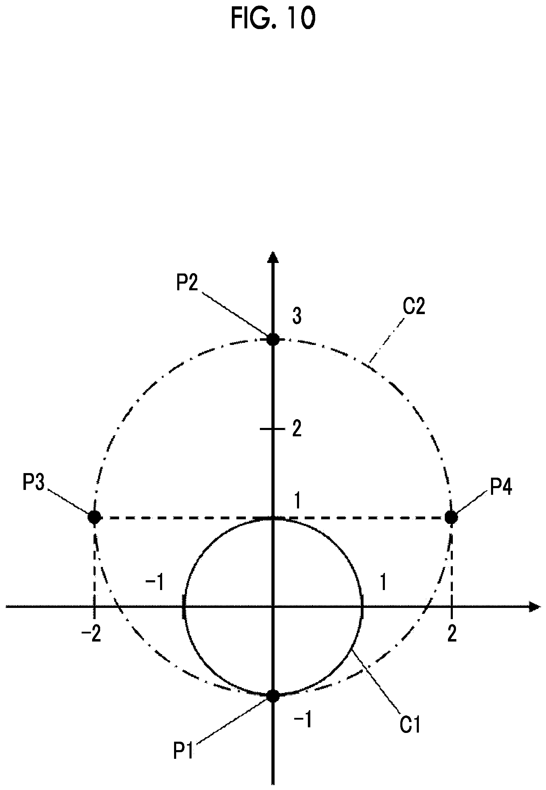

As a specific example, for example, as illustrated in Fig. 10, it is considered to approximate a circle C2 having four observation values, that is, a circle C2 passing through four points P1 to P4 using a circle C1 represented by (a, b, r) = (0, 0, 1) in the basic implicit function of Equation (7). The coordinates of the point P1 are (0, -1), the coordinates of the point P2 are (0, 3), the coordinates of the point P3 are (-2, 1), and the coordinates of the point P4 are (2, 1).

First, the initial value f(0, 0, 1) of the objective function f(a, b, r) is 96 from the four observation values. Assuming that the learning rate G is, for example, 0.01, the displacement amounts H1 to H3 are 0, 1.28, and 0.64 as indicated by Equations (12) to (14). These values are added to the initial values of a, b, and r, and 0, 1.28, and 1.64 are obtained as new values of a, b, and r. The value f(0, 1.28, 1.68) of the objective function f(a, b, r) in this case is 10.223, and a value smaller than the initial value f(0, 0, 1) is obtained. In this manner, by repeatedly performing the processing of deciding a, b, and r that reduce make the objective function f(a, b, r) smaller, the values of a, b, and r that minimize the objective function f(a, b, r) are decided, and the approximation curve of the organ having a circular shape is obtained.

$$-G \ \partial/\partial a \ f(0,0,1) = G \sum_{i=1}^{4} 4(x_i - a)\{(x_i - a)^2 + (y_i - b)^2 - r^2\} = 0 \cdot \cdot \cdot (1\,2)$$

$$-G \ \partial/\partial b \ f(0,0,1) = G \sum_{i=1}^{4} 4(y_i - b)\{(x_i - a)^2 + (y_i - b)^2 - r^2\} = 1.28 \cdot \cdot \cdot (1\,3)$$

$$-G \ \partial/\partial r \ f(0,0,1) = G \sum_{i=1}^{4} 4r \{(x_i - a)^2 + (y_i - b)^2 - r^2\} = 0.64 \cdot \cdot \cdot (1\,4)$$

In a case where the super-ellipse function indicated by Equation (1) is used as the basic implicit function for function approximation, the steepest descent method can be used which uses, as variables, the parameters of the super-ellipse, for example, the scale parameters W1 and W2 representing the scale of the super-ellipse, the shape parameter such as the shape parameter E representing the squareness of the super-ellipse, the shape parameter B representing the deflection of the super-ellipse, and the shape parameter T representing the balance of the super-ellipse, and the position parameter such as the position parameters L1 and L2 representing the arrangement position of the super-ellipse, and the position parameter A representing the rotation position of the super-ellipse. In this case, the shape parameters, position parameters and scale parameters which minimize the objective function are decided, and the implicit function approximating the organ is decided. The shape parameter W1/W2 or W2/W1 representing the aspect ratio of the super-ellipse is decided by optimizing the scale parameters W1 and W2.

In order for the user to grasp how much the finally obtained approximation curve is deformed from the curve represented by the basic implicit function used in a case of performing the function approximation, the shape analysis unit 36 displays the values of the shape parameters in the approximation function representing the finally obtained approximation curve, on the monitor 33. Further, in order to indicate how much the finally obtained approximation curve is deformed from the curve represented by the basic implicit function used in a case of performing the function approximation, the finally obtained approximation curve and the curve represented by the basic implicit function can be displayed on the monitor 33.

The organ evaluation unit 37 evaluates the disease state of the organ on the basis of the shape parameters of the approximation function represented by the implicit function approximated by the shape analysis unit 36. The organ evaluation unit 37 can evaluate the disease state of the organ by evaluating the shape of the organ using, as evaluation indexes, the shape parameters of the approximation function, such as the shape parameter W1/W2 or W2/W1 representing the aspect ratio of the approximation curve, the shape parameter E representing the squareness of the approximation curve, the shape parameter B representing the deflection of the approximation function, and the shape parameter representing the balance of the approximation curve, for example.

For example, as illustrated in Fig. 5, it is known that in a case where the prostate T2 is enlarged, the prostate T2 has a shape close to a circle. The organ evaluation unit 37 can evaluate whether or not there is a suspicion that benign prostatic hyperplasia has developed by evaluating the shape of the prostate of the subject on the basis of the shape parameter, for example. In this case, in a case where the shape parameter represented by the ratio W1/W2 or W2/W1 between the scale parameters W1 and W2 representing the scale of the approximation curve is close to 1, that is, has a value within a certain range around 1, the organ evaluation unit 37 can evaluate that there is a suspicion that the benign prostatic hyperplasia has developed, and in other cases, the organ evaluation unit 37 can evaluate that the possibility that the benign prostatic hyperplasia has developed is low. For example, in a case where the shape parameter E representing the squareness of the approximation curve is close to 1, that is, has a value within a certain range around 1, the organ evaluation unit 37 can evaluate that there is a suspicion that the benign prostatic hyperplasia has developed, and in other cases, the organ evaluation unit 37 can evaluate that the possibility that the benign prostatic hyperplasia has developed is low.

In a case where the shape parameter B representing the deflection of the approximation curve is smaller than a certain value, the organ evaluation unit 37 can evaluate that there is a suspicion that the benign prostatic hyperplasia has developed, and in other cases, the organ evaluation unit 37 can evaluate that the possibility that the benign prostatic hyperplasia has developed is low. Further, in a case where the shape parameter T representing the balance of the approximation curve has a value within a certain range close to 0, the organ evaluation unit 37 can evaluate that there is a suspicion that the prostate becomes enlarged, and in other cases, the organ evaluation unit 37 can evaluate that the possibility that the benign prostatic hyperplasia has developed is low.

As illustrated in Fig. 5, the enlarged prostate T2 protrudes toward the bladder R1, and the bladder R1 appears to have a shape with a recessed deep portion in the ultrasound image U. In such a case, it is generally known that cystitis tends to develop. In a case where the organ region extraction unit 35 detects the bladder R1 as the organ of the examination target, the shape analysis unit 36 can evaluate whether or not there is a suspicion that the cystitis has developed by evaluating the hollow shape of the bladder R1. In this case, for example, in a case where the shape parameter B representing the deflection in the approximation curve of the bladder R1 is greater than a certain value, the shape analysis unit 36 can evaluate that there is a suspicion that the cystitis has developed as the disease state of the bladder, and in other cases, the shape analysis unit 36 can evaluate that the risk that the cystitis has developed is low.

Further, for example, the organ evaluation unit 37 can indirectly evaluate the disease state of the organ by inputting the shape parameters to a machine learning model such as a so-called regression analysis model or a so-called classification tree model. In this case, the organ evaluation unit 37 can output an evaluation result for the organ by inputting the shape parameters of the approximation curve represented by the implicit function approximated by the shape analysis unit 36, to the machine learning model which has been trained using the relationship between the disease state of the organ and the shape parameters in advance, for example.

Under the control of the main body controller 39, the examination result memory 38 stores the ultrasound image U on which the processing of extracting the organ is performed by the organ region extraction unit 35, the extraction result of the organ by the organ region extraction unit 35, the information of the implicit function approximated by the shape analysis unit 36, and the evaluation result relating to the disease state of the organ by the organ evaluation unit 37, as the examination result in an association manner. The information of the implicit function approximated by the shape analysis unit 36 includes the shape parameters and the like of the implicit function. As the examination result memory 38, for example, recording media such as a flash memory, an HDD, an SSD, an FD, an MO disc, an MT, a RAM, a CD, a DVD, an SD card, and an USB memory can be used.

The processor 41 having the image generation unit 31, the display controller 32, the organ region extraction unit 35, the shape analysis unit 36, the organ evaluation unit 37, and the main body controller 39 is configured by a central processing unit (CPU) and a control program for causing the CPU to execute various kinds of processing, but the processor 41 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the image generation unit 31, the display controller 32, the organ region extraction unit 35, the shape analysis unit 36, the organ evaluation unit 37, and the main body controller 39 of the processor 41 can also be configured by being integrated partially or entirely into one CPU or the like.

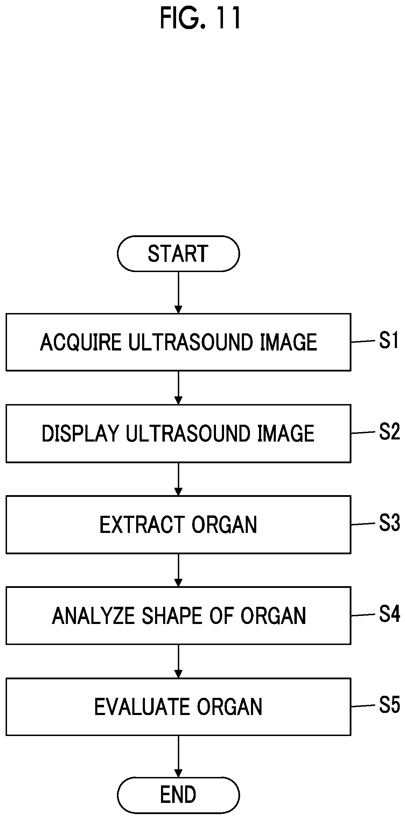

Next, the basic operation of the ultrasound diagnostic apparatus 1 according to the embodiment will be described using the flowchart illustrated in Fig. 11.

First, in Step S1, the user of the ultrasound diagnostic apparatus 1 brings the ultrasound probe 2 into contact with the body surface of the subject, and acquires the ultrasound image U in this state. In a case where the ultrasound image U1 is acquired, the transmission and reception circuit 22 performs so-called reception focusing processing under the control of the main body controller 39 to generate sound ray signals. The sound ray signals generated by the transmission and reception circuit 22 are sent to the image generation unit 31. The image generation unit 31 generates the ultrasound image U using the sound ray signals sent from the transmission and reception circuit 22. The ultrasound image U acquired in this manner is sent to the display controller 32, and is stored in the image memory 34.

In Step S2, the ultrasound image U which is acquired in Step S1 and is sent to the display controller 32 is subjected to predetermined processing, and then is displayed on the monitor 33. Thereby, the user can check the acquired ultrasound image U.

In Step S3, the organ region extraction unit 35 reads out the ultrasound image U acquired in Step S1 from the image memory 34, and extracts the organ of the subject by analyzing the ultrasound image U, under the control of the main body controller 39. In this case, for example, the organ region extraction unit 35 can extract the organ of the subject included in the ultrasound image U by applying a method of using simple pattern matching, a machine learning method described in Csurka et al.: Visual Categorization with Bags of Keypoints, Proc. of ECCV Workshop on Statistical Learning in Computer Vision, pp. 59-74 (2004) or a general image recognition method using deep learning or CNN described in Krizhevsk et al.: ImageNet Classification with Deep Convolutional Neural Networks, Advances in Neural Information Processing Systems 25, pp.1106-1114 (2012).

Further, the organ region extraction unit 35 can display the ultrasound image U on the monitor 33 by emphasizing the region of the extracted organ on the ultrasound image U. In this case, the organ region extraction unit 35 can display the region of the organ on the ultrasound image U in color, display a contour line of the region of the organ on the ultrasound image U, display a contour of the region of the organ on the ultrasound image U in color, and the like. The extraction result of the organ in Step S3 can be stored in the examination result memory 38.

In Step S4, the shape analysis unit 36 approximates the shape of the organ extracted in Step S3 using the implicit function. For example, the shape analysis unit 36 can set the x axis along the horizontal direction of the ultrasound image U, set the y axis along the direction orthogonal to the horizontal direction, and use the super-ellipse function indicated by Equation (1) as the implicit function. The shape analysis unit 36 can decide the implicit function of approximating the organ by using the steepest descent method which uses, as variables, the scale parameters W1 and W2 representing the scale of the super-ellipse, the shape parameter such as the shape parameter W1/W2 or W2/W1 representing the aspect ratio of the super-ellipse, the shape parameter E representing the squareness of the super-ellipse, the shape parameter B representing the deflection of the super-ellipse, and the shape parameter T representing the balance of the super-ellipse, and the position parameter such as the position parameters L1 and L2 representing the arrangement position of the super-ellipse, and the position parameter A representing the rotation position of the super-ellipse.

The value of the learning rate G used in the steepest descent method may be set in advance as initial setting, or may be input by the user via the input device 40. In a case where the learning rate G is input by the user, for example, the learning rate G may be input before the examination for the subject is started, or a message to prompt the user to input the learning rate G may be displayed on the monitor 33 in a case where Step S4 is started so that the learning rate G may be input by the user who has checked the message.

Further, the information including the shape parameter of the implicit function approximated in Step S4 can be stored in the examination result memory 38.

Finally, in Step S5, the organ evaluation unit 37 evaluates the disease state of the organ on the basis of the shape parameter of the approximation curve represented by the implicit function approximated in Step S4. The organ evaluation unit 37 can evaluate the disease state of the organ by using, as evaluation indexes, the shape parameters of the approximation function, such as the shape parameter W1/W2 or W2/W1 representing the aspect ratio of the approximation curve, the shape parameter E representing the squareness of the approximation curve, the shape parameter B representing the deflection of the approximation function, and the shape parameter representing the balance of the approximation curve, for example.

As illustrated in Fig. 5, it is known that in a case where the prostate T2 is enlarged, the prostate T2 has a shape close to a circle. Therefore, in a case where the shape parameter W1/W2 or W2/W1 representing the aspect ratio of the approximation curve has a value within a certain range around 1, in a case where the shape parameter E representing the squareness of the approximation curve has a value within a certain range around 1, in a case where the shape parameter B representing the deflection of the approximation curve is smaller than a certain value, or in a case where the shape parameter T representing the balance of the approximation curve has value within a certain range close to 0, the organ evaluation unit 37 can evaluate that there is a suspicion that the benign prostatic hyperplasia has developed, and in other cases, the organ evaluation unit 37 can evaluate that the possibility that the benign prostatic hyperplasia has developed is low.

As illustrated in Fig. 5, the enlarged prostate T2 protrudes toward the bladder R1, and the bladder R1 appears to have a shape with a recessed deep portion in the ultrasound image U. In such a case, it is generally known that cystitis tends to develop. In a case where the bladder R1 is detected in Step S3 as the organ of the examination target, the shape analysis unit 36 can evaluate the hollow shape of the bladder R1. In this case, for example, in a case where the shape parameter B representing the deflection in the approximation curve of the bladder R1 is greater than a certain value, the shape analysis unit 36 can evaluate that there is a suspicion that the cystitis has developed as the disease state of the bladder, and in other cases, the shape analysis unit 36 can evaluate that the risk that the cystitis has developed is low.

Further, for example, the organ evaluation unit 37 can evaluate the disease state of the organ by indirectly evaluating the shape parameters using the machine learning model such as a so-called regression analysis model or a so-called classification tree model. In this case, the organ evaluation unit 37 can output an evaluation result for the organ by inputting the shape parameters of the approximation curve represented by the implicit function approximated by the shape analysis unit 36, to the machine learning model which has been trained using the relationship between the disease state of the organ and the shape parameters in advance, for example.

The evaluation result relating to the organ of the subject obtained in this manner is displayed on the monitor 33 via the display controller 32. Further, the evaluation result relating to the organ of the subject is stored in the examination result memory 38. The evaluation result stored in the examination result memory 38 can be viewed by the user, for example, after the examination.

In a case where the processing of Step S5 is completed, the operation of the ultrasound diagnostic apparatus 1 according to the flowchart of Fig. 11 is ended.

Here, for example, in a case of examining an organ of which the shape is changed depending on the disease state, such as the prostate, usually, the user such as a doctor checks the ultrasound image U to perform an examination while qualitatively evaluating the shape of the organ shown in the ultrasound image U in many cases. However, in the qualitative evaluation, there are problems that it is difficult to evaluate a change in the shape of the organ in time series, and that accurate evaluation is difficult due to individual differences in the shape of the organ depending on the body type or the like of the subject.

With the ultrasound diagnostic apparatus 1 according to the embodiment of the present invention, the ultrasound image U is analyzed to extract the organ included in the ultrasound image U, the extracted organ is approximated using the implicit function, and the disease state of the organ is quantitatively evaluated on the basis of the shape parameters of the approximation curve approximated by the implicit function. Therefore, even in a case where there are individual differences in the shape of the organ depending on the body type or the like of the subject, the user can accurately evaluate the shape of the organ by checking the acquired evaluation result to accurately perform the examination. Further, in a case where the examination is regularly performed on the same subject using the ultrasound diagnostic apparatus 1, the user can easily evaluate the change of the shape of the organ in time series by checking the past examination result stored in the examination result memory 38 to more accurately perform the examination.

The description has been made in which one frame of the ultrasound image U is generated in Step S1 and the ultrasound image U is analyzed to extract the organ in Step S3, but in Step S1, a plurality of frames of ultrasound images U generated within a predetermined time of several seconds can be generated, and one frame of the ultrasound image U to be used for analysis can be selected from among the plurality of frames of ultrasound images U.

For example, in a case where the examination for the subject is started on the basis of an instruction from the user via the input device 40, the plurality of frames of ultrasound images U are generated within a predetermined time of several seconds from the examination start, and are stored in the image memory 34. The series of ultrasound images U stored in the image memory 34 in this manner are displayed on the monitor 33 in list display or so-called scroll display. The user can check the series of ultrasound images U displayed on the monitor 33, and select the ultrasound image U in which the organ as the examination target is most clearly shown, as the ultrasound image U to be used in the processing after Step S2. The selected ultrasound image U is displayed on the monitor 33 in Step S2, and is analyzed by the organ region extraction unit 35 in Step S3. In this manner, one frame of the ultrasound image U to be used in the processing after Step S2 is selected by the user so that the ultrasound image U in which the organ is more clearly shown can be processed, and thus the accuracy of the evaluation result of the disease state of the organ to be finally obtained by the organ evaluation unit 37 can be improved.

The organ region extraction unit 35 can analyze the series of ultrasound images U stored in the image memory 34 to automatically select the ultrasound image U to be used for extracting the organ. In this case, for example, the organ region extraction unit 35 can calculate the sharpness of the edge of the organ shown in each ultrasound image U of the series of ultrasound images U, and select one frame of the ultrasound image U with the highest calculated sharpness, as the ultrasound image U to be used in the subsequent processing. Here, for example, the organ region extraction unit 35 can detect the edge of the organ in the ultrasound image U, and calculate the sharpness on the basis of the contrast of the pixels around the detected edge. The ultrasound image U is automatically selected in this manner so that the ultrasound image U in which the organ is more clearly shown can be processed, and thus the accuracy of the evaluation result of the disease state of the organ to be finally obtained by the organ evaluation unit 37 can be improved.

The extraction result of the organ in Step S3 can be modified by the user via the input device 40, for example. For example, the organ extracted in Step S3 is superimposed on the ultrasound image U to be displayed on the monitor 33 in an emphasized manner, and in this case, the modification of the region of the organ by the user via the input device 40 is accepted. In a case where the user designates a region representing the organ on the ultrasound image U via the input device 40, the region of the organ extracted by the organ region extraction unit 35 is replaced with the region of the organ designated by the user. In this case, in Step S4 following Step S3, the region of the organ designated by the user is approximated using the implicit function. Thereby, even in a case where the organ region extraction unit 35 cannot correctly extract the organ for some reason, for example, in a case where the ultrasound image U is not sufficiently clear, it is possible to accurately evaluate the shape of the organ.

Further, the description has been made in which the transmission and reception circuit 22 is included in the ultrasound probe 2, but the transmission and reception circuit 22 can be included in the apparatus main body 3 instead of being included in the ultrasound probe 2.

Further, the description has been made in which the image generation unit 31 is included in the apparatus main body 3, but the image generation unit 31 may be included in the ultrasound probe 2 instead of being included in the apparatus main body 3.

The description has been made in which the ultrasound probe 2 and the apparatus main body 3 are connected in a wired manner, but the ultrasound probe 2 and the apparatus main body 3 may be connected in a wireless manner.

In the image generation unit 31, the description has been made in which the DSC 52 is connected to the signal processing unit 51 and the image processing unit 53 is connected to the DSC 52, but the image processing unit 53 may be connected to the signal processing unit 51, and the DSC 52 may be connected to the image processing unit 53. In this case, after the image processing unit 53 performs predetermined processing such as gradation processing on the ultrasound image U generated by the signal processing unit 51, the ultrasound image U is raster-converted by the DSC 52. As described above, even in a case where the signal processing unit 51, the image processing unit 53, and the DSC 52 are connected in this order, similarly to the case where the signal processing unit 51, the DSC 52, and the image processing unit 53 are connected in this order, the ultrasound image U is generated by the image generation unit 31.

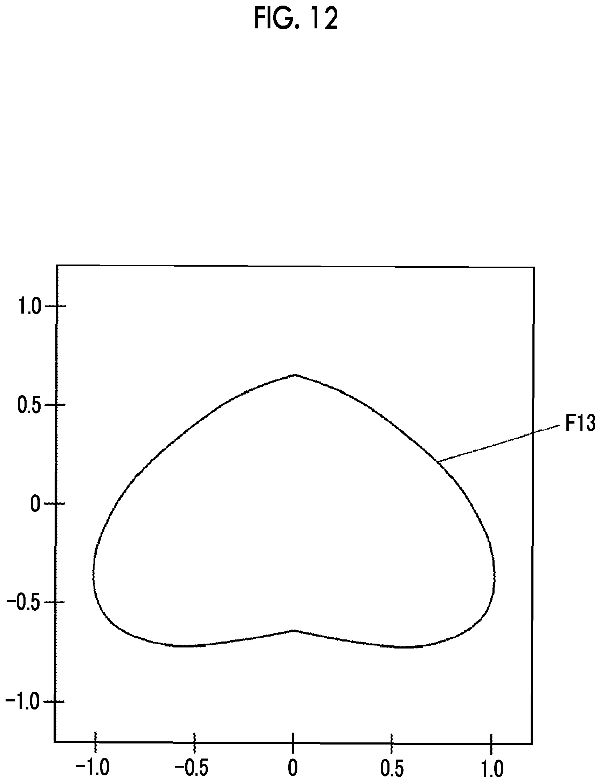

As the types of implicit functions used in a case where the shape analysis unit 36 performs the function approximation of the organ, a super-ellipse, an ellipse, and a circle are exemplified, but the implicit function to be used is not particularly limited thereto. For example, as the implicit function, a function indicated by the following Equation (15) can be used. For example, in a case where M = 1.1, N = 1.0, K = 0.4, and Q = 1.2 in Equation (15), a curve F13 illustrated in Fig. 12 is obtained. Equation (15) can be used as the implicit function in a case of approximating the shape of the prostate, for example, In a case where Equation (15) is used in the steepest descent method, for example, M, N, K, Q, and M/N that minimize the objective function using M, N, K, Q, and M/N as variables are decided.

$$(Nx)^2 + 2(My + K|x|^Q)^2 - 1 = 0 \cdot \cdot \cdot (1\ 5)$$

The shape analysis unit 36 can store in advance a plurality of basic implicit functions in a case of approximating the organ, such as the super-ellipse function of Equation (1), the circle function of Equation (7), and the function of Equation (15), and can set the basic implicit function used for approximating the organ according to the type of organ extracted by the organ region extraction unit 35. In this case, the shape analysis unit 36 can set the basic implicit function on the basis of the user's instruction via the input device 40. Further, the shape analysis unit 36 can store in advance a relationship between the types of plurality of organs and the basic implicit function corresponding to each organ, and automatically set the basic implicit function corresponding to the type of the organ extracted by the organ region extraction unit 35, on the basis of the stored relationship.

In this manner, by setting the basic implicit function according to the type of the organ, the organ is more accurately approximated using the implicit function, and the evaluation accuracy by the organ evaluation unit 37 can be improved.

The shape analysis unit 36 can divide the organ extracted by the organ region extraction unit 35 into a plurality of regions, and approximate the organ using the basic implicit function corresponding to each of the plurality of divided regions. For example, even in a case where the organ has a complicated shape, the shape analysis unit 36 can more accurately approximate the organ using the implicit function by combining the plurality of implicit functions in this manner.

The shape analysis unit 36 can recognize the shape of the organ extracted by the organ region extraction unit 35, and set an approximation method using the implicit function according to the recognized shape of the organ. For example, the shape analysis unit 36 can use the least squares method in a case of the shape of which the shape parameters are uniquely decided using the least squares method, such as a case where the shape of the organ extracted by the organ region extraction unit 35 is an ellipse shape or the like, and use the steepest descent method in other cases. Thereby, since an approximation method suitable for the shape of the organ is used, the organ is more accurately approximated, and the evaluation accuracy by the organ evaluation unit 37 can be improved.

Generally, in the steepest descent method, the objective function f(a, b, r) may not converge to a certain value depending on the value of the learning rate G. Thus, for example, in a case where the objective function f(a, b, r) becomes greater than a predetermined value, the shape analysis unit 36 can notify the fact to the user by displaying a message on the monitor 33 or the like, and prompt the user to change the value of the learning rate G. The user checks the notification from the shape analysis unit 36, and appropriately changes the value of the learning rate G, so that a, b, and r that minimize the objective function f(a, b, r) can be decided.

Further, the description has been made in which the processing such as the organ extraction by the organ region extraction unit 35 is performed on the ultrasound image U generated by the image generation unit 31, but the processing can be performed on the ultrasound image U that is stored in the image memory 34 in advance, such as the ultrasound image U stored in the image memory 34 in the past examination, instead of the ultrasound image U generated in the currently performed examination.

Although not illustrated, the ultrasound diagnostic apparatus 1 can comprise an external device connection circuit connected to an external device by wired connection or wireless connection, and the ultrasound image U input from the external device via the external device connection circuit can be stored in the image memory 34. In this case, for example, the processing such as the organ extraction by the organ region extraction unit 35 can be performed on the ultrasound image U input from the external device. As the external device for inputting the ultrasound image U, for example, an external ultrasound diagnostic apparatus, an external server device that stores the ultrasound image U, an external storage medium that stores the ultrasound image U are exemplified.

Further, the examination result stored in the examination result memory 38 via the external device connection circuit (not illustrated) can be output to the external device (not illustrated) such as a so-called workstation. Thereby, the user can check the examination result by using the external device after the examination is ended, for example.

The description has been made in which the present invention is applied to the ultrasound diagnostic apparatus 1 comprising the ultrasound probe 2, but the present invention can be applied to an image diagnostic apparatus not comprising the ultrasound probe 2, for example an image diagnostic apparatus configured by the apparatus main body 3 from which the image generation unit 31 is removed. In this case, the organ is extracted by the organ region extraction unit 35 on the basis of the ultrasound image U stored in advance in the image memory 34, the shape of the extracted organ is approximated using the implicit function by the shape analysis unit 36, and the disease state of the organ is evaluated by the organ evaluation unit 37 on the basis of the shape parameters of the approximation curve. The user can check the evaluation result of the disease state of the organ, which is obtained in the image diagnostic apparatus, and accurately evaluate the shape of the organ to accurately perform the examination.

The description has been made in which the organ extraction is performed for the ultrasound image U, the organ approximation is performed using the implicit function, and the disease state of the organ is evaluated on the basis of the shape parameters of the approximation curve represented by the approximate implicit function, but the same processing can be performed on any medical image such as a simple X-ray image, a computed tomography (CT) image, or a magnetic resonance imaging (MRI) image (magnetic resonance image) without being limited to the ultrasound image U. Therefore, the present invention can be applied to various diagnostic apparatuses which perform a diagnosis of any medical image, such as an X-ray image diagnostic apparatus, a CT image diagnostic apparatus, and an MRI image diagnostic apparatus, in addition to the ultrasound diagnostic apparatus 1.

Explanation of References

1: ultrasound diagnostic apparatus
2: ultrasound probe
3: apparatus main body
21: transducer array
22: transmission and reception circuit
23: pulser
24: amplification unit
25: AD conversion unit
26: beam former
31: image generation unit
32: display controller
33: monitor
34: image memory
35: organ region extraction unit
36: shape analysis unit
37: organ evaluation unit
38: examination result memory
39: main body controller

40: input device
41: processor
51: signal processing unit
52: DSC
53: image processing unit
C1, C2: circle
F1 to F13: curve
P1 to P4: point
R1: bladder
T1, T2: prostate
U: ultrasound image

**Claims**

1. A diagnostic apparatus (1) comprising:

   a monitor (33) that is configured to display an image in which an organ of a subject is imaged;
   an organ region extraction unit (35) that is configured to extract the organ by analyzing the image;
   a shape analysis unit (36) that is configured to approximate a shape of the organ extracted by the organ region extraction unit (35) using a super-ellipse function in a case where an x axis is set along a horizontal direction of the image and a y axis is set along a direction orthogonal to the horizontal direction; and
   an organ evaluation unit (37) that is configured to evaluate a disease state of the organ on the basis of a shape parameter of an approximation curve represented by the super-ellipse function approximated in the shape analysis unit (36);
   wherein the organ evaluation unit (37) is configured to evaluate the disease state of the organ using a shape parameter representing deflection of the approximation curve as an evaluation index or a shape parameter representing balance of a shape of the approximation curve as an evaluation index, wherein deflection of the approximation curve refers to deformation where end portions of the approximation curve in the horizontal direction along the x axis are pulled in a negative direction of the y axis.

2. The diagnostic apparatus (1) according to claim 1,

   wherein the organ is a prostate, and
   the organ evaluation unit (37) evaluates a disease state of the prostate by evaluating a shape of the prostate on the basis of the shape parameter.

3. The diagnostic apparatus (1) according to claim 1,

   wherein the organ is a bladder, and
   the organ evaluation unit (37) evaluates a disease state of the bladder by evaluating a hollow of the bladder on the basis of the shape parameter.

4. The diagnostic apparatus (1) according to any preceding claim,
   wherein the image is an ultrasound image.

5. The diagnostic apparatus (1) according to claim 4, further comprising:

   an ultrasound probe (2); and
   an image generation unit (31) that is configured to generate the ultrasound image by performing transmission and reception of ultrasound beams with respect to the subject using the ultrasound probe (2).

6. The diagnostic apparatus (1) according to any preceding claim,
   wherein the image is any one of an X-ray image, a computed tomography image, or a magnetic resonance image.

7. A control method of a diagnostic apparatus (1), the control method comprising:

   displaying an image in which an organ of a subject is imaged on a monitor;

extracting the organ by analyzing the image;
approximating a shape of the extracted organ using a super-ellipse function in a case where an x axis is set along a horizontal direction of the image and a y axis is set along a direction orthogonal to the horizontal direction; and
evaluating a disease state of the organ on the basis of a shape parameter of an approximation curve represented by the approximated super-ellipse function;
wherein evaluating a disease state of the organ comprises using a shape parameter representing deflection of the approximation curve as an evaluation index or a shape parameter representing balance of a shape of the approximation curve as an evaluation index, wherein deflection of the approximation curve refers to deformation where end portions of the approximation curve in the horizontal direction along the x axis are pulled in a negative direction of the y axis.

## Patentansprüche

1. Ein Diagnosegerät (1), das Folgendes umfasst:

   einen Monitor (33), der so konfiguriert ist, dass er ein Bild anzeigt, in dem ein Organ einer Person abgebildet ist;
   eine Organbereichsextraktionseinheit (35), die so konfiguriert ist, dass sie das Organ durch Analysieren des Bildes extrahiert;
   eine Formanalyseeinheit (36), die so konfiguriert ist, dass sie eine Form des Organs, das durch die Organbereichsextraktionseinheit (35) extrahiert wurde, unter Verwendung einer Super-Ellipsenfunktion in einem Fall annähert, in dem eine x-Achse entlang einer horizontalen Richtung des Bildes und eine y-Achse entlang einer Richtung orthogonal zu der horizontalen Richtung eingestellt ist; und
   eine Organbewertungseinheit (37), die so konfiguriert ist, dass sie einen Krankheitszustand des Organs auf der Grundlage eines Formparameters einer Näherungskurve bewertet, die durch die in der Formanalyseeinheit (36) angenäherte Super-Ellipsenfunktion dargestellt wird;
   wobei die Organbewertungseinheit (37) so konfiguriert ist, dass sie den Krankheitszustand des Organs unter Verwendung eines Formparameters, der die Auslenkung der Näherungskurve darstellt, als Bewertungsindex oder eines Formparameters, der das Gleichgewicht einer Form der Näherungskurve darstellt, als Bewertungsindex bewertet, wobei sich die Auslenkung der Näherungskurve auf eine Verformung bezieht, bei der die Endabschnitte der Näherungskurve in der horizontalen Richtung entlang der x-Achse in eine negative Richtung der y-Achse gezogen werden.

2. Das Diagnosegerät (1) nach Anspruch 1,

   wobei das Organ eine Prostata ist, und
   die Organbewertungseinheit (37) einen Krankheitszustand der Prostata bewertet, indem sie eine Form der Prostata auf der Grundlage des Formparameters bewertet.

3. Das Diagnosegerät (1) nach Anspruch 1,

   wobei das Organ eine Blase ist, und
   die Organbewertungseinheit (37) einen Krankheitszustand der Blase bewertet, indem sie einen Hohlraum der Blase auf der Grundlage des Formparameters bewertet.

4. Das Diagnosegerät (1) nach einem der vorhergehenden Ansprüche,
   wobei das Bild ein Ultraschallbild ist.

5. Das Diagnosegerät (1) nach Anspruch 4, ferner umfassend:

   eine Ultraschallsonde (2); und
   eine Bilderzeugungseinheit (31), die so konfiguriert ist, dass sie das Ultraschallbild durch Senden und Empfangen von Ultraschallstrahlen in Bezug auf die Person unter Verwendung der Ultraschallsonde (2) erzeugt.

6. Das Diagnosegerät (1) nach einem der vorhergehenden Ansprüche,
   wobei das Bild ein Röntgenbild, ein Computertomographie-Bild oder ein Magnetresonanzbild ist.

7.  Verfahren zur Steuerung eines Diagnosegeräts (1), wobei das Steuerungsverfahren umfasst:

    Anzeige eines Bildes, in dem ein Organ einer Person auf einem Monitor abgebildet ist;
    Extraktion des Organs durch Analyse des Bildes;
    Annäherung einer Form des extrahierten Organs unter Verwendung einer Super-Ellipsenfunktion in einem Fall, in dem eine x-Achse entlang einer horizontalen Richtung des Bildes und eine y-Achse entlang einer Richtung orthogonal zur horizontalen Richtung festgelegt ist; und
    Bewertung eines Krankheitszustands des Organs auf der Grundlage eines Formparameters einer Näherungskurve, die durch die angenäherte Super-Ellipsenfunktion
    dargestellt wird;
    wobei die Bewertung eines Krankheitszustands des Organs die Verwendung eines Formparameters, der die Ablenkung der Annäherungskurve darstellt, als Bewertungsindex oder eines Formparameters, der das Gleichgewicht einer Form der Annäherungskurve darstellt, als Bewertungsindex umfasst, wobei sich die Ablenkung der Annäherungskurve auf eine Verformung bezieht, bei der Endabschnitte der Annäherungskurve in der horizontalen Richtung entlang der x-Achse in eine negative Richtung der y-Achse gezogen werden.

## Revendications

1.  Appareil de diagnostic (1) comprenant :

    un moniteur (33) configuré pour afficher une image d'un organe d'un sujet ;
    une unité d'extraction de la région de l'organe (35) configurée pour extraire l'organe en analysant l'image ;
    une unité d'analyse de forme (36) configurée pour approximer la forme de l'organe extrait par l'unité d'extraction de région d'organe (35) à l'aide d'une fonction de super-ellipse dans le cas où un axe x est défini le long d'une direction horizontale de l'image et un axe y est défini le long d'une direction orthogonale à la direction horizontale ; et
    une unité d'évaluation de l'organe (37) configurée pour évaluer l'état pathologique de l'organe sur la base d'un paramètre de forme d'une courbe d'approximation représentée par la fonction de super-ellipse approximée dans l'unité d'analyse de forme (36) ;
    l'unité d'évaluation de l'organe (37) est configurée pour évaluer l'état pathologique de l'organe à l'aide d'un paramètre de forme représentant la déviation de la courbe d'approximation en tant qu'indice d'évaluation ou d'un paramètre de forme représentant l'équilibre d'une forme de la courbe d'approximation en tant qu'indice d'évaluation , la déviation de la courbe d'approximation faisant référence à une déformation où les parties terminales de la courbe d'approximation dans la direction horizontale le long de l'axe x sont tirées dans une direction négative de l'axe y.

2.  Appareil de diagnostic (1) selon la revendication 1,

    dans lequel l'organe est une prostate, et
    l'unité d'évaluation des organes (37) évalue un état pathologique de la prostate en évaluant la forme de la prostate sur la base du paramètre de forme.

3.  Appareil de diagnostic (1) selon la revendication 1,

    dans lequel l'organe est une vessie, et
    l'unité d'évaluation des organes (37) évalue un état pathologique de la vessie en évaluant une cavité de la vessie sur la base du paramètre de forme.

4.  Appareil de diagnostic (1) selon une des revendications précédentes,
    Dans lequel l'image est une image ultrasonique.

5.  Appareil de diagnostic (1) selon la revendication 4, comprenant en outre :

    une sonde à ultrasons (2) ; et
    une unité de génération d'images (31) configurée pour générer l'image ultrasonore en effectuant la transmission et la réception de faisceaux ultrasonores par rapport au sujet à l'aide de la sonde ultrasonore (2).

**6.** Appareil de diagnostic (1) selon une des revendications précédentes,
l'image est une image radiographique, une image tomodensitométrique ou une image par résonance magnétique.

**7.** Méthode de contrôle d'un appareil de diagnostic (1), la méthode de contrôle comprenant :

l'affichage d'une image dans laquelle un organe d'un sujet est représenté sur un moniteur ;
l'extraction de l'organe par l'analyse de l'image ;
approximation de la forme de l'organe extrait à l'aide d'une fonction de super-ellipse dans le cas où un axe x est défini le long d'une direction horizontale de l'image et un axe y est défini le long d'une direction orthogonale à la direction horizontale ; et
évaluer l'état pathologique de l'organe sur la base d'un paramètre de forme d'une courbe d'approximation représentée par la fonction super-ellipse approximée ;
dans lequel l'évaluation d'un état pathologique de l'organe comprend l'utilisation d'un paramètre de forme représentant la déviation de la courbe d'approximation comme indice d'évaluation ou d'un paramètre de forme représentant l'équilibre d'une forme de la courbe d'approximation comme indice d'évaluation, la déviation de la courbe d'approximation faisant référence à une déformation où les parties terminales de la courbe d'approximation dans la direction horizontale le long de l'axe x sont tirées dans une direction négative de l'axe y.

# FIG. 1

# FIG. 2

```
┌─────────────────────────────────┐
│        TRANSDUCER ARRAY         │──── 21
└─────────────────────────────────┘
```

TRANSMISSION AND
RECEPTION CIRCUIT ──── 22

```
┌──────────────────┐        ┌──────────────────┐
│      PULSER       │        │   AMPLIFICATION   │──── 24
│                  │        │       UNIT        │
└──────────────────┘        └──────────────────┘
        23                           │
                            ┌──────────────────┐
                            │  AD CONVERSION    │──── 25
                            │       UNIT        │
                            └──────────────────┘
                                     │
                            ┌──────────────────┐
                            │   BEAM FORMER     │──── 26
                            └──────────────────┘
```

# FIG. 3

IMAGE
GENERATION UNIT ──── 31

```
┌──────────────────┐
│      SIGNAL       │──── 51
│ PROCESSING UNIT   │
└──────────────────┘
         │
┌──────────────────┐
│       DSC         │──── 52
└──────────────────┘
         │
┌──────────────────┐
│      IMAGE        │──── 53
│ PROCESSING UNIT   │
└──────────────────┘
```

# FIG. 4

R1

33

T1

U

# FIG. 5

R1

33

T2

U

## FIG. 6

## FIG. 7

## FIG. 8

## FIG. 9

# FIG. 10

# FIG. 11

```
        ( START )
            │
            ▼
┌─────────────────────────────┐
│   ACQUIRE ULTRASOUND IMAGE   │──── S1
└─────────────────────────────┘
            │
            ▼
┌─────────────────────────────┐
│   DISPLAY ULTRASOUND IMAGE   │──── S2
└─────────────────────────────┘
            │
            ▼
┌─────────────────────────────┐
│       EXTRACT ORGAN         │──── S3
└─────────────────────────────┘
            │
            ▼
┌─────────────────────────────┐
│   ANALYZE SHAPE OF ORGAN     │──── S4
└─────────────────────────────┘
            │
            ▼
┌─────────────────────────────┐
│       EVALUATE ORGAN        │──── S5
└─────────────────────────────┘
            │
            ▼
        (  END  )
```

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016202208 A

- US 2011172533 A1

**Non-patent literature cited in the description**

- **CSURKA et al.** Visual Categorization with Bags of Keypoints. *Proc. of ECCV Workshop on Statistical Learning in Computer Vision,* 2004, 59-74

- **KRIZHEVSK et al.** ImageNet Classification with Deep Convolutional Neural Networks. *Advances in Neural Information Processing Systems,* 2012, vol. 25, 1106-1114
- **ZHANG, XIAOMING ; ROSIN, PAUL L.** Superellipse fitting to partial data. *Pattern Recognition,* 2003, vol. 36 (3), 743-752